(19) Europäisches Patentamt
European Patent Office
Office européen des brevets

(11) Publication number: **0 184 122 B1**

# (12) EUROPEAN PATENT SPECIFICATION

(45) Date of publication of patent specification: **17.07.91**

(51) Int. Cl.5: **C07D 251/16**, C07D 405/12, A01N 47/36

(21) Application number: **85115026.8**

(22) Date of filing: **27.11.85**

## (54) Sulfamoyl urea derivatives.

(30) Priority: **03.12.84 US 677617**
**04.02.85 US 689001**

(43) Date of publication of application:
**11.06.86 Bulletin 86/24**

(45) Publication of the grant of the patent:
**17.07.91 Bulletin 91/29**

(84) Designated Contracting States:
**BE DE FR GB IT**

(56) References cited:
**GB-A- 2 110 689**
**GB-A- 2 113 217**

**CHEMICAL ABSTRACTS, vol. 99, 1983, Columbus, OH (US); p. 563, no. 53793m**

(73) Proprietor: **PPG INDUSTRIES, INC.**
**One PPG Place**
**Pittsburgh Pennsylvania 15272(US)**

(72) Inventor: **van Gemert, Barry**
**7331 Shadyview Avenue**
**Massillon, OH 44646(US)**

(74) Representative: **Sternagel, Hans-Günther, Dr. et al**
**Patentanwälte Dr. Michael Hann Dr. H.-G. Sternagel Sander Aue 30**
**W-5060 Bergisch Gladbach 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

GB-A-2 110 689 and GB-A-2 113 217 describe herbicidally effective sulfamoyl urea derivatives in which the terminal N of the urea moiety carries a disubstituted triazine group and the terminal N of the sulfamoyl moiety carries a (di)substituted phenyl group and their herbicidally effective salts.

This invention relates to further herbicidally active sulfamoyl urea derivatives, including herbicidal formulations uses thereof to control the growth of noxious plants, i.e., weeds.

This invention concerns sulfamoyl urea derivatives represented by the Formula I:

I.

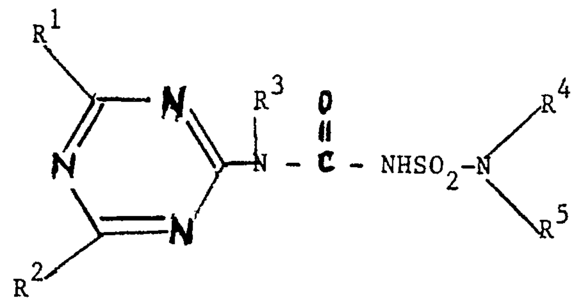

wherein:

$R^1$ and $R^2$

are the same or different and represent halogen or $C_1$ to $C_4$ alkyl, or alkoxy;

$R^3$ and $R^4$

are the same or different and represent hydrogen, $C_1$ to $C_4$ alkyl, alkoxyalkyl, haloalkyl, or up to $C_3$ alkenyl or alkynyl;

$R^5$ is:

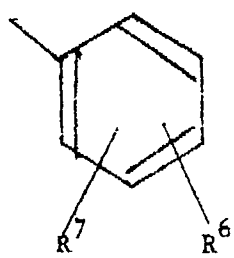

wherein:

$R^6$

is hydrogen or halogen;

$R^7$

is

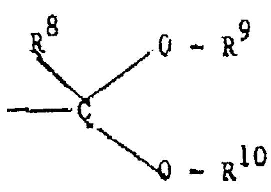

or

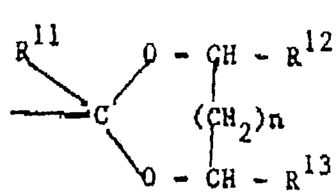

wherein:

$R^8$

is $C_1$ to $C_3$ alkyl;

$R^9$ and $R^{10}$

are $C_1$ to $C_6$ alkyl:

$R^{11}$
is $C_1$ to $C_3$ alkyl;
$R^{12}$ and $R^{13}$
are hydrogen or $C_1$ to $C_3$ alkyl; and
n is 0, 1 or 2 or agronomically suitable cationic salts of the Formula I compounds,

The Formula I compounds may conveniently be prepared by reacting a suitably substituted amino triazine of the Formula II:

II.

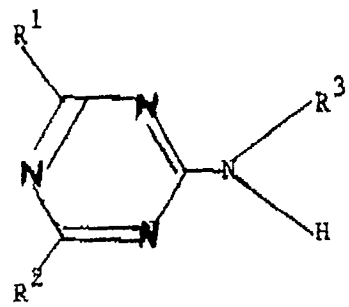

wherein $R^1$, $R^2$, $R^3$ are as previously defined, with a halosulfonyl isocyanate of the formula OCN-$SO_2$-Hal, wherein Hal is halogen, e.g., chlorine, fluorine or bromine to form the corresponding halosulfonyl urea of the Formula III:

III.

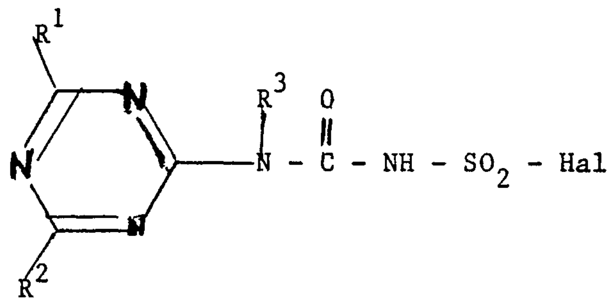

The Formula III compound is then reacted in the presence of an acid accepting agent with at least a stoichiometric amount of a suitably substituted amine of the Formula IV:

IV.

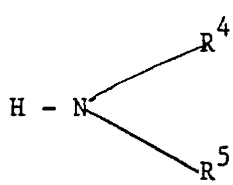

wherein $R^4$ and $R^5$ are as previously defined, to form a Formula I compound of the invention.

Preferred compounds of the invention are those wherein $R^3$, $R^4$ and $R^6$ are hydrogen. Preparation of particularly preferred compounds of the invention are illustrated by the following Examples:

Example I

Preparation of: 1-[2-(1,1-dimethoxyethyl)phenylsulfamoyl]-3-(4-methyl,6-methoxy-1,3,5-triazin-2-yl)urea

(a) To a flask was charged 6.0 grams of orthonitroacetophenone, 6.0 grams of trimethylorthoformate 0.25 gram of p-toluene sulfonic acid and 50 milliliters of methanol. The stirred mixture was then heated to 60° C. under a nitrogen blanket. After about 3 days at 60° C., HPLC indicated the reaction was over 90 percent complete. The mixture was cooled, poured into aqueous sodium carbonate solution, stripped of solvent and extracted with methylene chloride. The organic layer was dried over sodium carbonate and again stripped of solvent. The residue was dissolved in an aqueous solution of methanol containing 0.5 gram of sodium carbonate, transferred to a Parr shaker flask and reduced over 5% palladium on charcoal. The hydrogenated mixture was then filtered to remove catalyst. Most of the methanol was evaporated and the residue was extracted with methylene chloride. After phase separation, the organic layer was dried over anhydrous sodium carbonate and stripped of solvent affording a clear oil which was identified by NMR analysis as 2-(1,1-dimethoxyethyl)aniline.

(b) To 50 milliliters of methylene chloride maintained at 0-5° C., via an ice bath was added 1.38 grams of 4-methyl-6-methoxy-2-amino-1,3,5-triazine and 1.60 grams of chlorosulfonyl isocyanate. The mixture was stirred at ice temperature for about 3 hours after which a methylene chloride solution containing 1.10 grams of triethylamine and 1.70 grams of the amine prepared in part (a) of this Example was added. The mixture was permitted to rise to ambient temperature and washed with three portions of ice cold water and dried over anhydrous sodium sulfate. Filtration, followed by evaporation of solvent, afforded a pasty material which was dissolved in 80 milliliters of diethylether. The ether insoluble residue was discarded and the ether was evaporated, affording a resinous solid that was taken-up in pentane and filtered. Vacuum drying afforded 1.3 grams of yellow crystalline solid identified by NMR analysis as the desired product, 1-[2-(1,1-dimethoxyethyl)phenylsulfamoyl]-3-(4-methyl-6-methoxy-1,3,5-triazin-2-yl)urea.

Example II

Preparation of: 1-[2-(1,3-dioxolan-2yl-methyl)phenylsulfamoyl]-3-(4-methyl,6-methoxy-1,3,5-triazin-2-yl)urea

(a) To a round bottom flask was charged 10 grams of orthonitroacetophenone, 40 milliliters of ethylene glycol and 0.5 gram of p-toluene sulfonic acid. The stirred mixture was then heated to 140° C. with a nitrogen purge to remove water. After about 36 hours, HPLC indicated the reaction was over 90 percent complete. The mixture was cooled and suction filtered and the solid product was washed with aqueous sodium carbonate solution and suction dried affording 8 grams of white crystalline solid. The solid was taken up in a solution of methanol and aqueous sodium carbonate, transferred to a Parr shaker flask and 0.5 gram of palladium on charcoal was added. the mixture was hydrogenated at 50 psi for 2 hours, hydrogen uptake being complete at this point. The hydrogenated mixture was then filtered to remove catalyst and stripped of solvent on a rotary evaporator. The residue was then extracted with a mixture of water and methylene chloride. After phase separation, the organic phase was dried over anhydrous sodium sulfate and stripped on a rotary evaporator affording 7.0 grams of clear oil (which solidified on standing) which was identified by NMR analysis as 2-(1,3-dioxolan-2yl-methyl)aniline

(b) To 150 milliliters of methylene chloride maintained at 0-5° C. via an ice bath was added 1.41 grams (0.01 mole) of 4-methyl-6-methoxy-2-amino-1,3,5-triazine and 1.54 grams of chlorosulfonyl isocyanate. The mixture was stirred at ice temperature until all of the solid had dissolved and for an additional one-half hour after which a methylene chloride solution containing 0.01 mole of triethylamine and 0.01 mole of the amine prepared in part (a) of this Example were added dropwise, the mixture maintained at ice temperature throughout the addition. The mixture was permitted to rise to ambient temperature, recooled in the ice bath and poured into ice cold 2% aqueous hydrochloric acid where it was shaken and rapidly separated. The organic phase was washed with three portions of ice cold water and dried over anhydrous sodium sulfate. Evaporation of solvent afforded a pasty material that crystallized upon addition of a small amount of diethylether. Suction filtration afforded 2.6 grams of material identified by NMR analysis as the desired product, 1-[2-(1,3-dioxolan-2yl-methyl)phenylsulfamoyl]-3-(4-methyl-6-methoxy-1,3,5-triazin-2-yl)urea.

Although the invention has been illustrated by the foregoing Examples with regard to the preparation of specific compounds within the scope of Formula I, it is to be understood that other compounds within the scope of Formula I may readily be prepared by those skilled in the art simply by varying the choice of starting materials and using the same or similar techniques.

Weed control in accordance with this invention is effected by applying to the soil prior to emergence of weeds therefrom or to the plant surfaces subsequent to emergence from the soil, a herbicidally effective amount of a compound of this invention. It is, of course, to be understood that the term "a compound of this invention" also includes mixtures of such compounds or a formulation containing a compound or mixture of compounds of this invention.

The term "herbicidally effective amount" is amount of a compound of this invention required to so injure or damage weeds such that the weeds are incapable of recovering following application, while not causing any substantial damage to any valuable crop amongst which the weeds might be growing. The quantity of a compound of this invention applied in order to exhibit such satisfactory herbicidal effect may vary over a wide range and depends on a variety of factors, such as, for example, hardiness of a particular weed species, extent of weed infestation, climatic conditions, soil conditions, method of application, and the like. Typically, as little as 113.1 mg/m$^2$ or less (one or less pound per acre)of a compound of this invention would be expected to provide satisfactory weed control, although in some instances application rates in excess of 113.1 mg/m$^2$ (one pound per acre); e.g., up to 560.4 mg/m$^2$ or more ( 5 or more pounds per acre) might be required. Of course, the efficacy of a particular compound against a particular weed species may readily be determined by routine laboratory or field testing in a manner well known to the art. It is expected that satisfactory weed control can be had at a rate of application in the range of 1.12 mg/m$^2$ to 224.2 mg/m$^2$ ( 0.01 to 2.0 pounds per acre).

Of course, a compound of this invention can be formulated according to routine methods with any of several known and commonly used herbicidal diluents, adjuvants and carriers. The formulations can contain liquid carriers and adjuvants such as organic solvents, as well as emulsifiers, stabilizers, dispersants, suspending agents, spreaders, penetrants, wetting agents and the like. Typical carriers utilized in dry formulations include clay, talc, diatomaceous earth, silica and the like. Preferred formulations are those in the form of wettable powders, flowables, dispersible granulates or aqueous emulsifiable concentrates which can be diluted with water at the site of application. Also, dry formulations such as granules, dusts, and the like, may be used.

When desired, a compound of this invention can be applied in combination with other herbicidal agents in an effort to achieve even broader vegetative control. Typical herbicides which can be conveniently combined with Formula I compound include atrazine, hexazinone, metribuzin, ametryn, cyanazine, cyprazine, prometon, prometryn, propazine, simazine, terbutryn, propham, alachlor, acifluorfen, bentazon, metolachlor and N,N-dialkyl thiocarbamates such as EPTC, butylate or vernolate. These, as well as other herbicides described, for example, in the Herbicide Handbook of the Weed Science Society of America, may be used in combination with a compound or compounds of the invention. Typically such formulations will contain from about 5 to about 95 percent by weight of a compound of this invention.

The herbicidal formulations contemplated herein can be applied by any of several method known to the art. Generally, the formulation will be surface applied as an aqueous spray. Such application can be carried out by conventional ground equipment, or if desired, the sprays can be aerially applied. Soil incorporation of such surface applied herbicides is accomplished by natural leaching, and is of course facilitated by natural rainfall and melting snow. If desired, however, the herbicides can be incorporated into the soil by conventional tillage means.

Compounds of this invention are believed effective for preemergence or postemergence control of a wide variety of broadleaf and grassy weeds. Typical of the various species of vegetative growth that may be controlled, combated, or eliminated are, for example, annuals such as pigweed, lambsquarters, foxtail, crabgrass, wild mustard, field pennycress, ryegrass, goose grass, chickweed, wild oats, velvetleaf, purslane, barnyardgrass, smartweed, knotweed, cocklebur, kochia, medic, ragweed, hemp nettle, spurrey, pondweed, carpetweed, morningglory, ducksalad, cheatgrass, fall panicum, jimsonweed, witchgrass, watergrass, wild turnip, and similar annual grasses and weeds. Biennials that may be controlled include wild barley, campion, burdock, bull thistle, roundleaved mallow, purple star thistle, and the like. Also controlled by the compounds of this invention are perennials such as quackgrass, Johnsongrass, Canada thistle, curly dock, field chickweed, dandelion, Russian knapweed aster, horsetail, ironweed, sesbania, cattail, wintercress, horsenettle, nutsedge, milkweed, sicklepod, and the like.

More particularly, by way of example, the compounds prepared in the Examples were each tested for preemergence and postemergence herbicidal activity by spraying a solvent solution of same on a variety of common broadleaf and grassy weed species, under controlled laboratory conditions of light, temperature and humidity. The extent of herbicidal injury was evaluated, periodically after application, and a Numerical Injury Rating (NIR) assigned on a scale of from 0 (no injury) to 10 (all plants dead). The following Table gives the NIR assigned to each weed specie (identified by common name) 21 days after preemergence and postemergence application of the compound of Example I at a rate of 56.0 mg/m$^2$ (0.5 pound per acre):

| WEED SPECIES | PREEMERGENCE | POSTEMERGENCE |
|---|---|---|
| Teaweed | 9 | 5 |
| Jimsonweed | 9 | 10 |
| Wild mustard | 9 | 10 |
| Yellow nutsedge | 10 | 6 |
| Yellow foxtail | 10 | 8 |
| Large crabgrass | 9 | - |
| Johnsongrass | 9 | 8 |
| Coffeeweed | 9 | 8 |
| Velvetleaf | 9 | 8 |
| Tall morningglory | 9 | 3 |
| Wild oats | 8 | 3 |
| Barnyardgrass | 9 | 4 |

The following Table gives the NIR assigned to each weed species (identified by common name) 22 days after preemergence and postemergence application of the compound of Example II at rates of 56.0 mg/m$^2$ to 112.1 mg/m$^2$ (0.5 and 1.0 per acre):

| WEED SPECIES | PREEMERGENCE | | POSTEMERGENCE | |
|---|---|---|---|---|
| | 0.5 | 1.0 | 0.5 | 1.0 |
| Teaweed | 6 | 6 | 5 | 5 |
| Jimsonweed | 5 | 6 | 8 | 8 |
| Wild mustard | 8 | 8 | 8 | 10 |
| Yellow nutsedge | 10 | 10 | 6 | 7 |
| Yellow foxtail | 7 | 8 | 6 | 7 |
| Large crabgrass | 7 | 8 | - | - |
| Johnsongrass | 7 | 8 | 6 | 6 |
| Coffeeweed | 3 | 6 | 6 | 6 |

| | | | | |
|---|---|---|---|---|
| Velvetleaf | 5 | 5 | 8 | 8 |
| Tall morningglory | 4 | 5 | 5 | 6 |
| Wild oats | 8 | 7 | 2 | 1 |
| Barnyard grass | 8 | 8 | 3 | 5 |

## Claims

1. Sulfamoyl urea derivatives represented by the formula

(I) 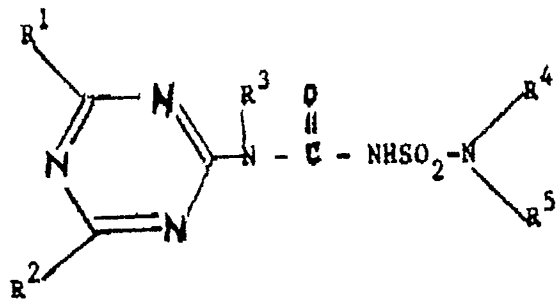

wherein:

$R^1$ and $R^2$
are the same or different and represent halogen or $C_1$ to $C_4$ alkyl, or alkoxy;

$R^3$ and $R^4$
are the same or different and represent hydrogen, $C_1$ to $C_4$ alkyl, alkoxyalkyl, haloalkyl, or up to $C_3$ alkenyl or alkynyl;

$R^5$
is

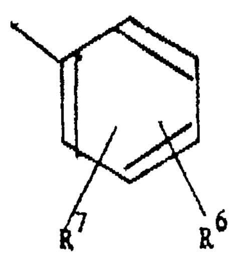

$R^6$
is hydrogen or halogen,

$R^7$
is

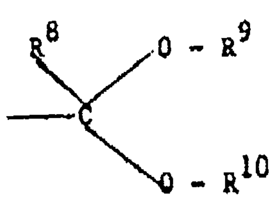

or

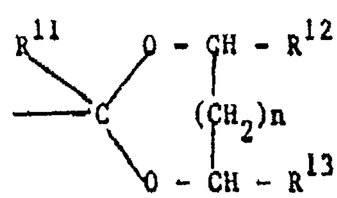

wherein:

$R^8$

is $C_1$ to $C_3$ alkyl;

$R^9$ and $R^{10}$

are $C_1$ to $C_6$ alkyl;

$R^{11}$

is $C_1$ to $C_3$ alkyl;

$R^{12}$ and $R^{13}$

are hydrogen or $C_1$ to $C_3$ alkyl, and

n is 0, 1 or 2

or the agronomically suitable salts of the Formula I compounds.

2. The sulfamoyl urea derivatives of claim 1 wherein $R^3$, $R^4$ and $R^6$ are hydrogen.

3. The sulfamoyl urea derivatives of claim 2 that is 1-[2-(1,1-dimethoxyethyl)phenylsulfamoyl)-3-(4-methyl-6-methoxy-1,3,5-triazin-2yl)urea or 1-[2-(1,3-dioxolan-2yl-methyl)phenylsulfamoyl]-3-(4-methyl-6-methoxy-1,3,5-triazin-2yl)urea.

4. A herbicidal formulation containing an agronomically acceptable carrier and a derivative or mixture of derivatives defined in claim 1.

5. Use of a herbicidal formulation comprising a derivative or mixture of derivatives of claim 1 for controlling weeds wherein a herbicidally effective amount of herbicide is applied to a growth medium prior to emergence of weeds therefrom or to the weeds subsequent to their emergence from the growth medium.

## Revendications

1. Dérivés de sulfamoyl-urée représentés par la formule

(I)

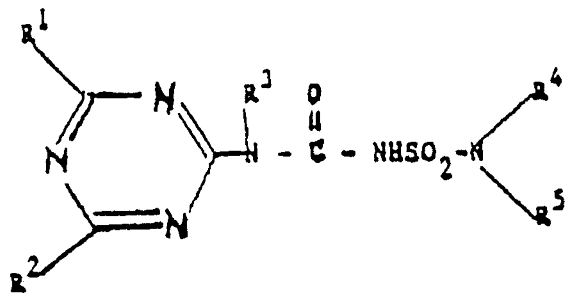

dans laquelle :

$R^1$ et $R^2$

sont identiques ou différents et représentent un halogène ou un alkyle de $C_1$ à $C_4$ ou un alkoxy

$R^3$ et $R^4$

sont identiques ou différents et représentent un hydrogène, un alkyle de $C_1$ à $C_4$, un alkoxyalkyle, un haloalkyle, ou un alkényle ou un alkynyle jusqu'à $C_3$ ;

$R^5$

est :

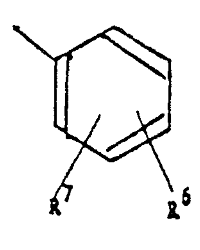

$R^6$
est un hydrogène ou un halogène ;
$R^7$
est

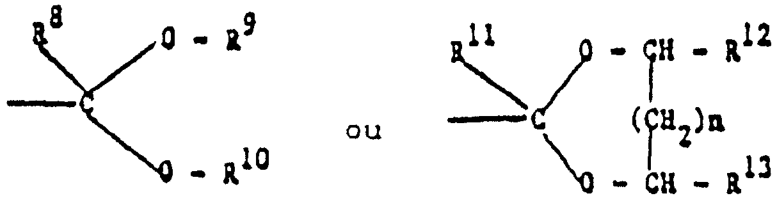

dans laquelle :
$R^8$
est un alkyle de $C_1$ à $C_3$ ;
$R^9$ et $R^{10}$
sont alkyle de $C_1$ à $C_6$ ; $R^{11}$
est un alkyle de $C_1$ à $C_3$ ;
$R^{12}$ et $R^{13}$
sont hydrogène ou alkyle de $C_1$ à $C_3$ ; et n est 0, 1 ou 2
ou les sels appropriés à l'agronomie, des composés de la Formule I.

2. Dérivés de sulfamoyl-urée de la revendication 1 où $R^3$, $R^4$ et $R^6$ sont hydrogène.

3. Dérivés de sulfamoyl-urée de la revendication 2 qui sont 1-[2-(1,1 - diméthoxyéthyl) phénylsulfamoyl] - 3 - (4-méthyl-6-méthoxy - 1,3,5 - triazine - 2yl) urée ou 1- [2-(1,3 - dioxolan-2yl-méthyl) phénylsulfamoyl] - 3-(4 - méthyl - 6 - méthoxy -1,3,5 - triazine - 2yl) urée.

4. Formulation herbicide contenant un véhicule acceptable agronomiquement et un dérivé ou un mélange de dérivés définis dans la revendication 1.

5. Utilisation d'une formulation herbicide comprenant un dérivé ou un mélange de dérivés de la revendication 1 pour lutter contre les plantes adventices, dans laquelle une quantité efficace d'herbicide est appliquée à un milieu où se développent des plantes adventices avant leur levée ou sur celles-ci après leur levée du milieu de croissance.

**Patentansprüche**

1. Sulfamoylharnstoffderivate der Formel

(I)

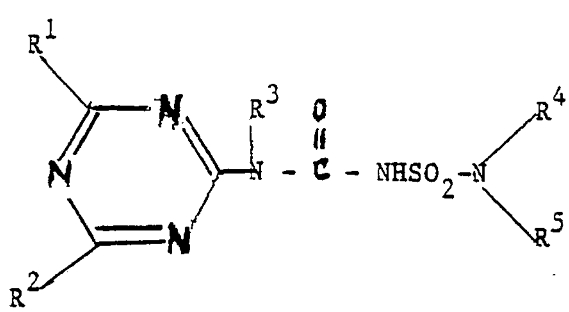

in der
$R^1$ und $R^2$
gleich oder unterschiedlich sind und Halogen oder $C_1$- bis $C_4$-Alkyl oder Alkoxy sind,
$R^3$ und $R^4$

gleich oder unterschiedlich sind und Wasserstoff, $C_1$- bis $C_4$-Alkyl, Alkoxyalkyl , Haloalkyl oder bis zu $C_3$-Alkenyl oder Alkynyl sind,
$R^5$
ist

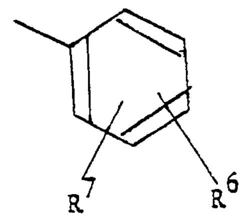

$R^6$
ist Wasserstoff oder Halogen,
$R^7$
ist

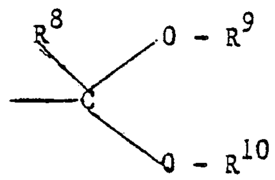

oder

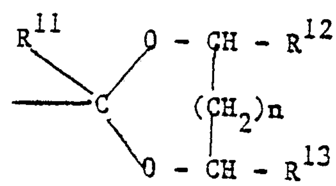

in denen
$R^8$
$C_1$- bis $C_3$-Alkyl ist,
$R^9$ und $R^{10}$
$C_1$- bis $C_6$-Alkyl sind,
$R^{11}$
$C_1$- bis $C_3$-Alkyl ist,
$R^{12}$ und $R^{13}$
Wasserstoff oder $C_1$- bis $C_3$-Alkyl sind, und
n 0, 1 oder 2 ist,
oder die landwirtschaftlich nutzbaren Salze der Verbindungen der Formel I.

2. Sulfamoylharnstoffderivate nach Anspruch 1,
**dadurch gekennzeichnet,**
daß $R^3$, $R^4$ und $R^6$ Wasserstoff sind.

3. Sulfamoylharnstoffderivate nach Anspruch 2,
**dadurch gekennzeichnet,**
daß sie 1-[2(1,1-Dimethoxyethyl)phenylsulfamoyl]-3-(4-methyl-6-methoxy-1,3,5-triazin-2yl)harnstoff oder 1-[2-(1,3-Dioxolan-2yl-methyl)phenylsulfamoyl]-3-(4-methyl-6-methoxy-1,3,5-triazin-2yl)harnstoff sind.

4. Herbizidformulierung, enthaltend einen landwirtschaftlich anwendbaren Träger und ein Derivat oder Mischung von Derivaten, wie in Anspruch 1 angegeben.

5. Verwendung einer Herbizidformulierung, enthaltend ein Derivat oder Mischung von Derivaten nach Anspruch 1 zum Steuern des Wachstums von Unkräutern, wobei eine herbizidwirksame Menge des Herbizids auf ein Wachstumsmedium vor dem Auflaufen der Unkräuter oder auf die Unkräuter nach ihrem Auflaufen aus dem Wachstumsmedium aufgebracht wird.